# FASCICULE DE BREVET EUROPEEN

(11) **EP 2 462 879 B1**
(45) Date de publication et mention de la délivrance du brevet: **20.11.2019**
(21) Numéro de dépôt: 11191882.7
(22) Date de dépôt: 05.12.2011
(51) Int. Cl.: A61B 17/072, A61B 17/115, A61B 17/00

(54) **Dispositif médical de renfort**
Medizinische Vorrichtung zur Aussteifung
Medical reinforcement device

(30) Priorité: 08.12.2010 FR 1060273
(43) Date de publication de la demande: 13.06.2012
(73) Titulaire: Les Laboratoires Brothier, 92000 Nanterre (FR)
(72) Inventeur: Girardiere, Christian, 75116 Paris (FR); Barikosky, Michel, 75007 Paris (FR)
(74) Mandataire: Gevers & Orès

(56) Documents cités:
- FR-A1- 2 789 885
- US-A1- 2005 059 996
- US-A1- 2006 085 034
- US-A1- 2010 147 923

## Description

La présente invention se rapporte à un dispositif médical de renfort de zones tissulaires suturées.

L'invention trouve particulièrement application dans le domaine des anastomoses mécaniques, notamment en chirurgie viscérale.

L'anastomose est un acte chirurgical manuel ou mécanique qui consiste à rétablir la connexion entre deux conduits anatomiques. Cet acte est fortement utilisé en chirurgie viscérale, ou digestive.

Les anastomoses digestives désignent l'opération qui consiste, après résection (coupure) d'une partie du tube digestif, à rétablir une continuité en assemblant les deux bouts 101,102 du tube digestif restants. Ce type d'intervention peut être réalisé par exemple dans le cas de cancer colorectal, mais également de pathologies inflammatoires de type maladie de Crohn, rectocolites hémorragiques, diverticulite etc.

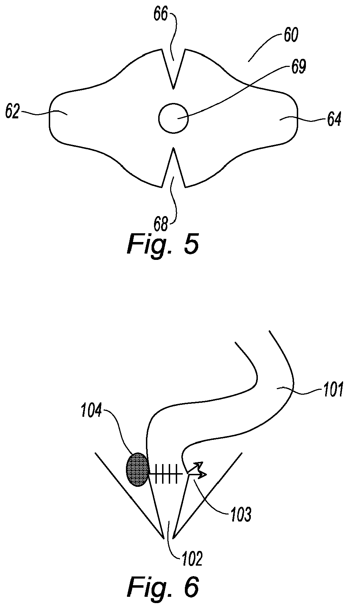

Suite à ce type d'intervention, des fuites 103 peuvent survenir au niveau de l'anastomose et entraîner de graves complications qui vont retarder la guérison du patient au mieux ou entraîner des infections 104 au pire (figure 6).

Les anastomoses peuvent être réalisées manuellement avec du fil de suture ou mécaniquement avec des agrafeuses.

L'utilisation ces dernières années de techniques mécaniques utilisant des pinces d'agrafage linéaires et circulaires, a à la fois facilité le geste du chirurgien et la sécurité du patient en diminuant notamment l'apparition de fistules. Toutefois, le risque de complications comme des fuites ou une sténose et le taux de mortalité restent significatifs. Afin de diminuer ces complications et ce taux de mortalité, de nouvelles stratégies ont été mises en place avec l'utilisation de dispositifs de renfort d'agrafages afin de réduire les saignements et diminuer les tensions sur les tissus adjacents. Certains de ces dispositifs renforts sont résorbables mais d'autres ne le sont pas. Des exemples de dispositifs de renfort peuvent comprendre des dispositifs en PTFE (PolyTétraFluoroEthylène), des dispositifs de péricarde bovin, de polymères résorbables à base de cellulose ou de tissus intestinaux porcins.

L'état de la technique comprend notamment les documents US-A1-2005/005996 et US-A1-2010/0147923.

Cependant, ces dispositifs présentent de nombreux inconvénients notamment un prix élevé, une innocuité virale incertaine ainsi que des risques dûs aux prions.

La présente invention vise à améliorer la situation en proposant un dispositif de renfort permettant de diminuer les risques liés à l'utilisation de pinces d'agrafage lors des anastomoses mécaniques.

A cet effet, un premier aspect de la présente invention concerne un dispositif médical de renfort pour zone tissulaire suturée comportant une ligne d'agrafage et un cercle d'agrafage, le dispositif ayant une forme d'anneau, pour recouvrir et renforcer la zone le long du cercle d'agrafage, dispositif caractérisé par le fait que l'anneau comporte des moyens latéraux de recouvrement et de renforcement, pour recouvrir et renforcer des portions de la ligne d'agrafage s'étendant au-delà du cercle d'agrafage.

De manière remarquable, grâce à ses moyens de recouvrement et de protection latéraux, le dispositif de renfort du premier aspect de la présente invention est particulièrement adapté aux anastomoses mécaniques utilisant un double agrafage linéaire et circulaire. La forme annulaire du dispositif permet le recouvrement de la zone d'agrafage circulaire et les moyens latéraux permettent le recouvrement et le renforcement des portions de zone d'agrafage linéaire hors de la zone circulaire.

On connaît, dans l'état de la technique, des dispositifs de renfort ayant une forme d'anneau et munis de languettes adhésives. Ces languettes adhésives sont exclusivement destinées à fixer le dispositif de renfort sur une pince mécanique d'agrafage circulaire. Une fois que le dispositif de renfort est positionné sur la zone suturée, les languettes sont détachées du dispositif de renfort par pivotement de l'agrafeuse dans l'anastomose immobilisée, puis la pince est retirée. Ces dispositifs n'ont donc rien à voir avec celui de la présente demande.

Selon une réalisation préférée, les moyens de recouvrement et de protection latéraux sont des ailettes de recouvrement et de renforcement.

Par ailleurs, on sait que les anastomoses mécaniques entraînent un risque de sténose, c'est-à-dire de rétrécissement, post-opératoire plus élevé que les anastomoses manuelles. Ce risque de sténose est également dépendant des zones anatomiques à agrafer, des antécédents du patient et de la réalisation de l'anastomose proprement dite, notamment de la tension plus ou moins importante de l'anastomose. Le traitement de la sténose consiste habituellement en une ou plusieurs dilatations mécaniques par bougie ou par ballonnet jusqu'à ce que la sténose cède.

L'utilisation d'un dispositif de renfort rend malheureusement ces dilatations plus difficiles à mener en raison de la rigidification de la zone suturée renforcée.

La présente divulgation vise à améliorer la situation en proposant un dispositif de renfort n'ayant pas cet inconvénient.

A cet effet, un deuxième aspect non revendiqué concerne un dispositif médical de renfort pour zone tissulaire suturée comportant un cercle d'agrafage, le dispositif ayant une forme d'anneau, pour recouvrir et renforcer la zone le long du cercle d'agrafage, dispositif caractérisé par le fait que l'anneau comporte au moins une encoche située dans une partie périphérique de l'anneau et agencée pour être disposée au droit du cercle d'agrafage.

Grâce à cette encoche, on forme ainsi une discontinuité dans le renforcement de la zone suturée rendant cette zone plus souple. Cet assouplissement permet de manière remarquable de faciliter le traitement par dilatation en cas de sténose.

De préférence, l'encoche a une forme en U ou en V ou en C couché, qui, à la périphérie du dispositif de renfort, écarte ses bords.

Avantageusement, une telle encoche peut être prévue sur le dispositif de renfort du premier aspect de la présente invention. Cela permet d'avoir un dispositif de renfort efficace adapté aux anastomoses utilisant un double agrafage linéaire et circulaire sur une zone tissulaire présentant un risque élevé de sténose.

Les dispositifs de renfort selon les premier et deuxième aspects permettent tous les deux de résoudre le même problème de diminuer le risque de fuites dans une zone suturée lors d'une anastomose mécanique. Plus particulièrement, le dispositif de renfort muni de moyens latéraux du premier aspect de l'invention permet de diminuer le risque de fuites dans le cadre d'une anastomose par double agrafage linéaire et circulaire. De son côté, le dispositif de renfort muni d'encoches du deuxième aspect non revendiqué apporte une souplesse fort utile au praticien, notamment pour faciliter un traitement par dilatation en cas d'apparition d'une sténose post-opératoire, pour une anastomose par simple agrafage circulaire ou pour une anastomose par double agrafage linéaire et circulaire.

La présente invention offre ainsi un dispositif de renfort minimisant les risques de fuite lors d'une anastomose mécanique. Les variantes de réalisation du premier aspect de l'invention et du deuxième aspect non revendiqué seront choisies par le chirurgien en fonction de la zone tissulaire à agrafer (à risque plus ou moins élevé de sténose) et de la technique de réalisation de l'anastomose (simple ou double agrafage).

Avantageusement, le dispositif de renfort est formé d'un textile en alginate de calcium. Cela permet d'améliorer la cicatrisation des zones tissulaires reconnectées en renforçant, par voie naturelle, après résorption du dispositif, l'anastomose et ainsi d'éviter l'apparition de fuites et de fistules au niveau de l'ensemble des sutures.

Selon une autre caractéristique, le dispositif est conformable tout en présentant une mémoire de forme.

Ces propriétés peuvent être obtenues en utilisant un textile en alginate de calcium enduit au moyen d'un polymère biocompatible et biodégradable, par exemple l'alginate de propylène glycol (PGA).

Ces propriétés permettent au dispositif d'épouser les contours des zones tissulaires afin d'assurer un contact optimal et de garantir ainsi une bonne étanchéité et une bonne migration des agents hémostatiques et cicatrisants.

En outre, ces caractéristiques de mise en forme et de mémoire de forme permettent une utilisation du dispositif en coelioscopie.

Des modes de réalisation de l'invention vont maintenant être décrits de façon plus précise mais non limitative en regard des dessins annexés sur lesquels :
- la figure 1 illustre un dispositif de renfort selon le premier aspect de l'invention ;
- la figure 2 illustre les différentes étapes (en vues latérales et de dessus) d'une anastomose par double agrafage utilisant le dispositif de renfort de la figure 1 ;
- la figure 3 illustre un dispositif de renfort selon le deuxième aspect non revendiqué ;
- la figure 4 illustre l'état du dispositif de renfort de la figure 3 après un agrafage circulaire ;
- la figure 5 illustre un dispositif de renfort combinant les caractéristiques du dispositif de la figure 1 et du dispositif de la figure 3 ; et
- la figure 6 illustre le problème des fuites anastomotiques.

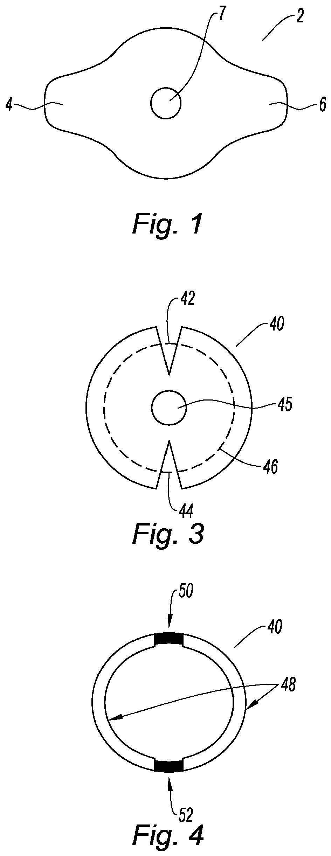

La figure 1 représente un dispositif de renfort 2 ayant une forme d'anneau plan comportant des ailettes latérales 4,6 et un trou circulaire central 7.

Le dispositif de renfort 2 biorésorbable est formé d'un textile en alginate de calcium, de préférence enduit sur l'une de ses deux faces d'une solution d'un polymère biocompatible et biodégradable comme par exemple l'alginate de propylène glycol (PGA).

L'activité hémostatique et cicatrisante des alginates de calcium est en effet reconnue dans le domaine de la cicatrisation des plaies. Le taux de calcium d'une part, et sa vitesse de libération d'autre part, sont des paramètres clés permettant de contrôler l'hémostase et la cicatrisation.

Un alginate de type guluronique est préféré à un alginate riche en motif mannuronique car ce dernier libère rapidement les ions calciums et ne permet pas de maîtriser les étapes biologiques menant à la guérison des tissus.

Selon une réalisation préférée, le textile du dispositif de renfort 2 est un tricot obtenu à partir d'un fil d'alginate de calcium dont le titre est choisi entre 100 et 500 dTex (décitex). Le rapport molaire entre les deux monomères de base : acide mannuronique M et acide guluronique G est choisi tel que M/G est inférieur à 1. Cela crée une surface non lisse possédant une porosité contrôlée, de préférence supérieure à 75 µm. Une telle porosité permet de garantir une forte réaction fibroblastique menant à l'intégration tissulaire du matériau et donc à l'adhérence du dispositif de renfort 2 à une zone tissulaire. Cette réaction est due à la pénétration des macrophages, des fibroblastes et des fibres de collagène. A l'inverse une surface de porosité inférieure à 10 µm génère peu d'adhérence. De préférence, la maille du tricot est un jersey bloqué de type « locked stitches » avec une jauge 32 possédant un diamètre inter-maille supérieur à 75 µm.

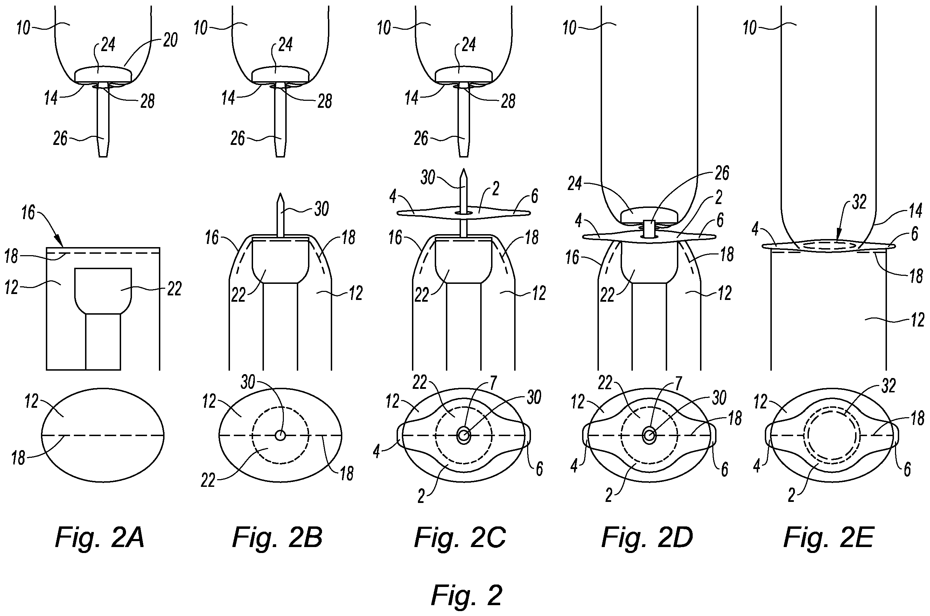

Les figures 2A à 2E de la figure 2 illustrent les différentes étapes d'une anastomose par double agrafage, par exemple colorectale, utilisant le dispositif de renfort 2.

La figure 2A illustre une partie colique 10 et une partie rectale 12 d'une zone tissulaire situées de part et d'autre d'une partie digestive « malade », par exemple présentant une tumeur, réséquée en une extrémité colique 14 et en une extrémité rectale 16. La partie colique 10 est située du côté du colon et la partie rectale 12 est située du côté du rectum.

La résection à l'extrémité colique 14 est réalisée de manière conventionnelle au bistouri froid alors que la résection à l'extrémité rectale 16 est réalisée à l'aide d'une agrafeuse linéaire coupante, non représentée, de sorte qu'une ligne d'agrafage 18 est formée.

Suite à la résection de la partie « malade », un agrafage circulaire est réalisé à l'aide d'une agrafeuse circulaire coupante comprenant une enclume 20 et un corps de pince 22. L'enclume 20 comprend une tête 24, de préférence flexible, et une tige 26.

Pour cela, l'enclume 20 est introduite dans la partie colique 10 depuis l'extrémité colique 14. Elle est fixée au niveau de cette extrémité colique par un fil 28 serrant l'extrémité colique 14 autour de la tige 26.

Ensuite, le corps de pince 22 est introduit, par exemple par voie rectale, dans la partie rectale 12.

Une vue de dessus de la partie rectale 12 est illustrée en bas de la figure 2A, dans laquelle on distingue bien la ligne d'agrafage 18.

L'étape suivante, illustrée sur la figure 2B, consiste en une perforation de l'extrémité rectale 16 réalisée en sortant un trocart 30 du corps de pince 22. C'est la pointe du trocart qui perfore l'extrémité rectale 16. Le résultat de cette perforation est illustré sur la vue de dessus de la partie rectale 12 en bas de la figure 2B.

Après perforation de l'extrémité rectale 16, le dispositif de renfort 2 est introduit sur le trocart 30 de la manière représentée à la figure 2C. Afin d'assurer le maintien du dispositif de renfort 2 sur le trocart 30, le diamètre du trou 7 est choisi sensiblement égal, de préférence inférieur au diamètre du trocart 30.

Une vue de dessus de la partie rectale 12 à l'issue de cette introduction du dispositif de renfort est représentée en bas de la figure 2C.

La figure 2D illustre l'étape suivante consistant à rapprocher la tige 26 de l'enclume 20 et le trocart 30 pour réaliser un agrafage circulaire.

Après encliquetage de la tige 26 et du trocart 30, suivi d'un agrafage circulaire, un cercle d'agrafage, désigné par la référence 32 sur la figure 2E est alors formé. La partie du dispositif de renfort 2, la partie circulaire de l'extrémité colique 14 et la partie circulaire de l'extrémité rectale 16 délimitées par le cercle d'agrafage 32 sont simultanément découpées.

La dernière opération effectuée par le chirurgien consiste alors à retirer le corps de pince 22 et l'enclume 20. Le retrait de l'enclume 20 est facilité grâce au choix de la tête 24 flexible.

Le résultat final de ce double agrafage linéaire circulaire est illustré sur la vue de dessus de la partie rectale 12 en bas de la figure 2E.

De manière remarquable, la zone tissulaire suturée est entièrement couverte par le dispositif de renfort 2, les ailettes latérales 4 permettant un recouvrement parfait des portions de la ligne d'agrafage 18 restantes après le découpage de la partie du dispositif de renfort 2 délimitée par le cercle d'agrafage 32. Ainsi, le risque de fuites postopératoires est minimisé.

La figure 3 illustre un dispositif de renfort 40 selon un deuxième aspect non revendiqué. Le dispositif 40 a une forme d'anneau plan comportant deux encoches 42, 44 périphériques et un trou circulaire central 45.

Avantageusement, le dispositif de renfort 40 est formé du même textile en alginate de calcium que le dispositif de renfort 2 et possède de ce fait les mêmes propriétés de biorésorbabilité, de protection et de cicatrisation d'une zone tissulaire que le dispositif de renfort 2.

Le dispositif de renfort 40 de la figure 3 est adapté à être utilisé lors d'une anastomose mécanique par simple agrafage circulaire, par exemple une anastomose duodéno-jéjunale, une anastomose gastro-duodénale, une anastomose oeso-gastrique, une anastomose colo-colique, etc. Une telle anastomose est réalisée de la même manière que l'anastomose illustrée aux figures 2A à 2E à l'exception de l'étape d'agrafage linéaire.

A l'issue d'un tel agrafage circulaire créant une zone tissulaire suturée comprenant un cercle d'agrafage 46, le dispositif de renfort 40 a la forme représentée à la figure 4. Ce dispositif recouvre au niveau des zones périphériques 48 une grande portion du cercle d'agrafage 46 résultant de l'anastomose diminuant ainsi le risque de fuites postopératoires.

De manière remarquable, grâce aux encoches 42 et 44 disposées au droit du cercle d'agrafage 46, deux zones étroites de discontinuité 50 et 52 apparaissent, au niveau desquelles il n'y aucun recouvrement de zone tissulaire. Ces zones de discontinuité du recouvrement, beaucoup plus souples que les zones périphériques 48 rigidifiées en raison du renforcement du dispositif 40, permettent de faciliter un traitement de dilatation ultérieur en cas de sténose post-opératoire.

En outre, la présence de ces zones de discontinuité facilite le retrait de l'enclume après l'agrafage circulaire, rendant l'utilisation du dispositif de renfort 40 optimale même avec une enclume n'ayant pas une tête flexible.

La figure 5 décrit une variante de réalisation du dispositif de renfort selon l'invention combinant les caractéristiques des dispositifs de renfort 2 et 40.

Un tel dispositif de renfort 60 a une forme d'anneau plan. Il comporte deux ailettes latérales 62, 64, deux encoches 66,68 périphériques agencées pour être disposées au droit du cercle d'agrafage et un trou circulaire central 69.

Il est particulièrement adapté aux anastomoses par double agrafage linéaire et circulaire de zones tissulaires à risque de sténose élevé.

Bien entendu, d'autres modes de réalisation encore peuvent être envisagés.

Ainsi, un nombre d'encoches différent de deux peut être prévu, par exemple une seule encoche ou encore un nombre d'encoches supérieur à deux.

On a parlé plus haut des caractéristiques de mise en forme et de mémoire de forme du dispositif de renfort de l'invention. Il s'agit de caractéristiques très intéressantes. En effet, le dispositif de renfort doit pouvoir être utilisé en coelioscopie, ce qui nécessite le passage du dispositif par le trocart. Le dispositif sera donc replié sur lui-même. Après passage dans le trocart, il est essentiel que le dispositif retrouve sa forme initiale pour l'agrafage avec l'enclume de l'agrafeuse.

## Revendications

1. Dispositif médical (2) de renfort pour zone tissulaire suturée comportant une ligne d'agrafage (18) et un cercle d'agrafage (32), le dispositif (2) ayant une forme d'anneau, pour recouvrir et renforcer la zone le long du cercle d'agrafage (32), dispositif (2) **caractérisé par le fait que** l'anneau comporte des moyens latéraux de recouvrement et de renforcement, pour recouvrir et renforcer des portions de la ligne d'agrafage (18) s'étendant au-delà du cercle d'agrafage (32).

2. Dispositif (2) selon la revendication 1, **caractérisé par le fait que** les moyens latéraux sont des ailettes (4,6) de recouvrement et de renforcement.

3. Dispositif selon la revendication 1 ou 2, **caractérisé par le fait que** l'anneau comporte au moins une encoche (66,68) située dans une partie périphérique de l'anneau et agencée pour être disposée au droit du cercle d'agrafage.

4. Dispositif selon la revendication 3, **caractérisé par le fait qu'**il comporte deux encoches (42,44 ; 66,68).

5. Dispositif selon l'une quelconque des revendications précédentes, **caractérisé par le fait qu'**il est formé d'un textile en alginate de calcium.

6. Dispositif selon l'une quelconque des revendications précédentes, **caractérisé par le fait qu'**il est conformable et présente une mémoire de forme.

## Patentansprüche

1. Medizinische Vorrichtung (2) zur Aussteifung für einen genähten Gewebebereich, umfassend eine Klammerlinie (18) und einen Klammerkreis (32), wobei die Vorrichtung (2) eine Ringform aufweist, um den Bereich entlang des Klammerkreises (32) abzudecken und auszusteifen, wobei die Vorrichtung (2) durch die Tatsache gekennzeichnet ist, dass der Ring seitliche Abdeck- und Aussteifungsmittel umfasst, um Abschnitte der Klammerlinie (18), die sich über den Klammerkreis (32) hinaus erstrecken, abzudecken und auszusteifen.

2. Vorrichtung (2) nach Anspruch 1, **gekennzeichnet durch** die Tatsache, dass die seitlichen Mittel Abdeck- und Aussteifungsflügel (4,6) sind.

3. Vorrichtung nach Anspruch 1 oder 2, **gekennzeichnet durch** die Tatsache, dass der Ring mindestens eine Kerbe (66,68) umfasst, die sich in einem Umfangsteil des Rings befindet und angeordnet ist, um auf gleicher Höhe mit dem Klammerkreis bereitgestellt zu sein.

4. Vorrichtung nach Anspruch 3, **gekennzeichnet durch** die Tatsache, dass sie zwei Kerben (42,44; 66,68) umfasst.

5. Vorrichtung nach einem der vorstehenden Ansprüche, **gekennzeichnet durch** die Tatsache, dass sie aus einem Textil aus Calciumalginat ausgeformt ist.

6. Vorrichtung nach einem der vorstehenden Ansprüche, **gekennzeichnet durch** die Tatsache, dass sie anpassbar ist und ein Formgedächtnis aufweist.

## Claims

1. Medical reinforcement device (2) for a tissue region with sutures comprising a staple line (18) and a staple circle (32), the device (2) having the shape of a ring to cover and reinforce the region along the staple circle (32), device (2) **characterised by** the fact that the ring comprises lateral means for covering and reinforcing, to cover and reinforce portions of the staple line (18) extending beyond the staple circle (32).

2. Device (2) according to claim 1, **characterised by** the fact that the lateral means are covering and reinforcement fins (4,6).

3. Device according to claim 1 or 2, **characterised by** the fact that the ring comprises at least one notch (66,68) located in a peripheral part of the ring and arranged to be placed at the right angle of the staple circle.

4. Device according to claim 3, **characterised by** the fact that it comprises two notches (42,44; 66,68).

5. Device according to any one of the preceding claims, **characterised by** the fact that it is formed of a calcium alginate textile.

6. Device according to any one of the preceding claims, **characterised by** the fact that it can be shaped and has shape-memory properties.
